# EUROPÄISCHE PATENTSCHRIFT

(11) **EP 3 281 654 B1**
(45) Veröffentlichungstag und Bekanntmachung des Hinweises auf die Patenterteilung: **03.04.2019**
(21) Anmeldenummer: 17184562.1
(22) Anmeldetag: 02.08.2017
(51) Int. Cl.: A61M 1/10

(54) **PERISTALTIKPUMPE MIT ROTATORISCHEM SPIEL**
PERISTALTIC PUMP WITH ROTATIONAL PLAY
POMPE PÉRISTALTIQUE AYANT UN JEU DE ROTATION

(30) Priorität: 11.08.2016 DE 102016114959
(43) Veröffentlichungstag der Anmeldung: 14.02.2018
(73) Patentinhaber: B. Braun Avitum AG, 34212 Melsungen (DE)
(72) Erfinder: SCHÄFER, Oliver, 36286 Neuenstein (DE); ISKE, Andreas, 34320 Söhrewald (DE)
(74) Vertreter: Winter, Brandl, Fürniss, Hübner, Röss, Kaiser, Polte - Partnerschaft mbB

(56) Entgegenhaltungen:
- US-A- 4 432 707
- US-A- 6 041 709
- US-A1- 2005 238 515
- US-A1- 2007 148 022

## Beschreibung

### Hintergrund der Erfindung

Die Erfindung betrifft eine Peristaltikpumpe für eine Vorrichtung zur extrakorporalen Blutbehandlung, insbesondere für eine Dialysemaschine, zum Fördern von Fluid in der Vorrichtung, wobei die Peristaltikpumpe einen mit einer Antriebswelle drehangetriebenen Rotor aufweist, der mit einer elastisch/flexibel verformbaren Fluidleitung eine Querschnittsverengung ausbildend zusammenwirkt, wobei die Querschnittsverengung zur Fluidförderung durch Rotation des Rotors entlang der Fluidleitung deplatziert (fortbewegt) wird und wobei der Rotor mit der Antriebswelle mittels einer Koppelstruktur drehmomentübertragend gekoppelt ist. Sie betrifft des Weiteren eine Vorrichtung zur extrakorporalen Blutbehandlung mit einer erfindungsgemäßen Peristaltikpumpe, insbesondere eine Dialysemaschine, mit einer solchen Peristaltikpumpe.

Eine Peristaltikpumpe hat in einer Vorrichtung zur extrakorporalen Blutbehandlung, zum Beispiel in einer Dialysemaschine, die Aufgabe ein definiertes Volumen eines Mediums, wie zum Beispiel Blut oder Dialysefluid, durch Verformen und Abklemmen einer elastisch verformbaren Fluidleitung von einer negativen Druckseite (Niederdruckseite) auf eine positive Druckseite (Hochdruckseite) zu fördern. Bekannte Peristaltikpumpen bei solchen Medizingeräten weisen in der Regel einen Rotor, ein Pumpengehäuse und die zwischen Rotor und Pumpengehäuse angeordnete elastische Schlauchleitung als Fluidleitung auf. Der Rotor ist drehmomentfest mit einer Antriebsachse der Maschine gekoppelt, durch diese angetrieben und trägt in radialer Richtung positionierbare und mittels Druckfedern gegen die Schlauchleitung und Pumpengehäuse vorgespannte Anpresselemente, z.B. in Form von Anpressrollen.

### Stand der Technik

Eine derartige Pumpe, bei der Anpressrollen mittels Blattfedern nach radial außen vorgespannt sind, ist beispielsweise aus der US 3,737,256 bekannt. Bei dieser Pumpe ist der Rotor über einen Kupplungsmechanismus mit einer Antriebsachse verbunden und an dieser in Axialrichtung mittels einer Schraube gesichert. Bei dieser Pumpe ist ein Nachteil, dass ein Lösen/Auswechseln des Rotors nur unter Zuhilfenahme eines Schraubwerkzeugs und unter entsprechendem zeitlichem Aufwand erfolgen kann.

Aus der WO 97/28368 A2 ist eine peristaltische Pumpe für eine Dialysemaschine bekannt, bei der mittels Spiralfedern radial nach außen vorgespannte Anpressrollen eines Rotors mit einer Quetschleitung zusammenwirken. Der Rotor weist eine zentrale Aufnahmeöffnung für eine Antriebswelle eines Antriebsmotors auf und ist auf dieser mittels eines schwenkbaren und formschlüssig mit der Antriebswelle zusammenwirkenden Handgriffs gekoppelt. Der Handgriff ist einerseits in eine Koppelstellung schwenkbar, in der er formschlüssig mit der Antriebswelle zusammenwirkt, und andererseits in eine Betätigungsstellung, in der er von der Antriebswelle entkoppelt ist und eine Art Kurbel für eine Handbetätigung des Rotors ausbildet.

Aus der US 4,527,323 ist eine peristaltische Pumpe für eine Dialysemaschine bekannt, deren Rotor ähnlich zu dem der US 3,737,256 ausgebildet ist und mit einem Zapfen axial in einen Schlitz einer Antriebsachse geschoben wird, um Rotor und Antriebsachse miteinander zu koppeln. Das Patent offenbart des Weiteren einen auf den Rotor aufsetzbaren Handgriff zur manuellen Montage oder Betätigung des Rotors.

Bei bekannten Systemen und insbesondere bei den vorstehend beschriebenen Pumpen ist es generell von Nachteil, dass es bei Pumpen zu einer erhöhten Beanspruchung der Antriebskomponenten und damit erhöhtem Verschleiß kommen kann, wenn es beim Betrieb zu Lastwechseln oder gar Lastumkehrungen kommt. Dies kann insbesondere der Fall sein, wenn der Rotor lösbar mit einer Antriebsachse verbunden bzw. verbindbar ist, zum Beispiel mittels radialem Formschluss oder Kraftschluss. Derartige Lastwechsel oder Lastumkehrungen können anwendungsfallbedingt auftreten und zu einer variierenden oder gar wechselnden Beanspruchung von Komponenten des Antriebs zum Beispiel durch Last- oder Drehmomentumkehr führen. Eine Begleiterscheinung solcher Lastvariationen kann eine erhöhte Geräuschemission sein.

Besonders können solche Lastvariationen bei peristaltisch arbeitenden Schlauchrollenpumpen auftreten, bei denen konstruktionsbedingt immer ein Einlaufbereich und ein Auslaufbereich existieren, die im Allgemeinen auch als Einlaufgeometrie bzw. Auslaufgeometrie bezeichnet werden. Der Einlaufbereich und der Auslaufbereich sind dadurch bedingt, dass das eingelegte Schlauchsystem des extrakorporalen Blutkreislaufs über ein offenes Pumpenschlauchsegment verfügt und es sich nicht um ein vollumfänglich geschlossenes Schlauchsegment handelt. Der Rotor steht daher nur über einen bestimmten Winkelbereich mit dem Schlauchsegment in Eingriff und quetscht dieses in Interaktion mit dem Pumpengehäuse zusammen, während er in einem verbleibenden Winkelbereich außerhalb des Eingriffs vom Schlauchsegment gelöst ist. Zwischen den beiden Winkelbereichen liegt jeweils der Einlaufabschnitt, in dem der Rotor mit dem Schlauchsegment in Eingriff gelangt, bzw. der Auslaufabschnitt, in dem sich der Rotor aus dem Eingriff mit dem Schlauchsegment löst. Beim Auslaufen des Rotors aus der Auslaufgeometrie kommt es zu einer kurzen Drehmomentvariation oder sogar zu einer Drehmomentumkehr, da die zur Kompression des Pumpensegmentes/Schlauchsegments erforderliche Kompressionsfeder des Rotors das beim Auslaufen neu entstehende radiale Spiel zur Dekompression nutzt und den Rotor relativ zur Rotation der Antriebswelle (zusätzlich zu deren Geschwindigkeit) vorlaufen lässt, bis die Antriebswelle das Spiel wieder eingeholt hat. Aufgrund von Toleranzen zwischen der Antriebswelle und der mit dieser zusammenwirkenden Koppelstruktur des Rotors kann es so zu unerwünschten nachteilhaften Krafteinleitungen auf Zahnflanken der Getriebeeinheit und zu nachteiligen und unerwünschten akustischen Effekten kommen.

Die US 2007/0148022 A1 zeigt eine Peristaltikpumpe, in welcher ein Schlauch um einen kompletten Umfang eines Rotors gelegt ist, wodurch keine durch die Elastizität des Schlauchs und dessen Wechselwirkungen mit Rollern des Rotors bedingte Lastumkehrungen auftreten. Allerdings ist hierfür ein komplexer Mechanismus vorgesehen, um die Pumpe montieren zu können und um den Schlauch in einer Ruhestellung entlasten zu können.

Auch die in US 2005/0238515 A1 zeigt eine aus mehreren Peristaltikpumpen zusammengesetzte Pumpenvorrichtung, in welcher zwei Schlauchabschnitte einander diametral gegenüberliegend eingesetzt sind, wodurch auch hier keine entsprechenden Lastwechsel auftreten. In dieser Vorrichtung ist eine komplexe Antriebswelle mit Mitnehmern notwendig, um die Rotoren der einzelnen Peristaltikpumpen unterschiedlich zueinander ausrichten zu können, um dadurch lokale Verhärtungen des Schlauchs zu vermeiden.

In US 6 041 709 A ist eine Peristaltikpumpe eines Tintendruckers offenbart, wobei in dieser Druckschrift der Schlauch und die Roller der Rotoren derart angeordnet sind, dass stets gleichzeitig ein Roller mit dem Schlauch in Eingriff kommt und ein Roller außer Eingriff mit dem Schlauch kommt.

Aus der US 4 432 707 A ist eine Peristaltikpumpe für Waschchemikalien offenbart, bei welcher der Rotor durch ein Loslager in Axialrichtung beweglich auf der Antriebswelle sitzt, um fertigungs- und betriebsbedingte Varianzen zu kompensieren.

### Kurzbeschreibung der Erfindung

Ausgehend von dem vorstehend beschriebenen Stand der Technik liegt der vorliegenden Erfindung die Aufgabe zugrunde, die zuvor angeführten Nachteile zu beseitigen, insbesondere eine Peristaltikpumpe für eine Vorrichtung zur extrakorporalen Blutbehandlung, insbesondere für eine Dialysemaschine, bereit zu stellen, die eine höhere Verschleißbeständigkeit und eine geringere Geräuschbelastung aufweist.

Gemäß der vorliegenden Erfindung wird diese Aufgabe gelöst durch eine Peristaltikpumpe für eine Vorrichtung zur extrakorporalen Blutbehandlung, insbesondere für eine Dialysemaschine, zum Fördern von Fluid in der Vorrichtung, wobei die Peristaltikpumpe einen mit einer Antriebsachse oder Antriebswelle drehangetriebenen Rotor aufweist, der mit einer elastisch/flexibel verformbaren Fluidleitung eine Querschnittsverengung ausbildend zusammenwirkt, die zur Fluidförderung durch Rotation des Rotors entlang der Fluidleitung weiterbewegt wird, wobei der Rotor mit der Antriebswelle oder Antriebsachse mittels einer Koppelstruktur (Kupplung) drehmomentübertragend gekoppelt ist, wobei die Koppelstruktur den Rotor und die Antriebswelle mit einem Spiel in Rotationsrichtung zueinander koppelt. Dabei weist die Koppelstruktur zumindest eine parallel zur Rotationsachse ausgebildete ebene erste Führungskontur sowie zumindest eine zweite Führungskontur auf, welche zum Zusammenwirken mit der ersten Führungskontur ausgebildet und bestimmt, wobei die zweite Führungskontur abschnittsweise oder auch vollständig gerundet ausgebildet ist, um eine Abrollbewegung der ersten Führungskontur auf einem Radius der zweiten Führungskontur bei einer Relativrotation im Spielbereich zu bewirken.

Die Aufgabe wird außerdem gelöst durch eine Vorrichtung zur extrakorporalen Blutbehandlung mit einer erfindungsgemäßen Peristaltikpumpe, insbesondere wie in der vorliegenden Beschreibung offenbart oder wie in den angehängten Ansprüchen beansprucht.

Der vorliegende Erfindungsgegenstand lässt sich auch so definieren, dass die Erfindung in einer Erhöhung der radialen Freiheitsgrade eines radialen Verriegelungselementes (als ein Bestandteil der erfindungsgemäßen Koppelstruktur) besteht, wodurch eine bei bekannten Pumpen vorliegende unerwünschte negative Krafteinleitung auf die Antriebswelle und mit dieser zusammenwirkenden Zahnflanken einer vorgeschalteten Getriebeeinheit verringert/verhindert werden. Des Weiteren können durch die Erfindung bei bekannten Pumpen auftretende akustische Effekte (Klacken), die durch Kraftvariationen/Momentvariationen beim Passieren des Ein- und/oder Auslaufbereichs hervorgerufen werden, verringert sowie eine Verbesserung der Getriebelebensdauer erzielt werden.

Die Koppelstruktur ermöglicht durch ihre Geometrie eine Art "Freilauf" innerhalb eines durch die Koppelstruktur definierten Winkelbereiches. Dieser kann insbesondere empirisch ermittelt worden sein. Der ausgewählte/empirisch ermittelte Winkelbereich ist in der Regel abhängig von Design und Materialeigenschaften der einzelnen Funktionselemente der Peristaltikpumpe, wie zum Beispiel (nämlich) des Rotors und der Antriebswelle, sowie von jeweils angewandten/definierten Betriebsparametern (z.B. Drehzahl). Das durch die Koppelstruktur erzeugte Spiel in Rotationsrichtung kann nach der Erfindung grundsätzlich im Auslaufbereich des Rotors aus dem Pumpensegment und/oder im Einlaufbereich des Rotors in das Pumpensegment wirken bzw. seinen erfindungsgemäß zugedachten Effekt erzielen.

Wie vorstehend mit Bezug auf den Stand der Technik beschrieben wurde, kann im Einlaufbereich und/oder im Auslaufbereich eine Variation/Schwankung oder gar Umkehr von auf Druckelemente des Rotors wirkenden Radialkräften (oder von auf den Rotor wirkenden Momenten/Umfangskräften) auftreten, die sich auch auf die Antriebswelle und einen mit dieser wirkverbundenen Antriebsstrang (Getriebe) auswirken könnten. Bei einem Auslaufen des Rotors, insbesondere eines mit der Fluidleitung zusammenwirkenden Anpresselements (Fluidleitungs-Stützstruktur), aus dem die Fluidleitung zusammenquetschenden Abschnitt kann zum Beispiel ein zusätzliches Drehmoment insbesondere in Rotationsrichtung hervorgerufen werden. Dieses wäre von der Antriebswelle aufzunehmen und zu kompensieren und würde sich somit hinsichtlich akustischer Emissionen (Klackgeräusche) und Belastung der Antriebswelle und deren Getriebe negativ auswirken. Das erfindungsgemäß vorgesehene Spiel der Koppelstruktur kann derartige Variationen/Schwankungen ausgleichen oder zumindest mindern, indem der Rotor einen relativen Vorlauf bzw. einen relativen Nachlauf gegenüber der Antriebswelle haben kann. Trotz des vorgesehenen Spiels sind Rotor und Antriebswelle miteinander drehmomentübertragend gekoppelt.

Durch die erfindungsgemäße Freilauffunktion (infolge des in Rotationsrichtung wirkenden Spiels der Koppelstruktur) kann insbesondere auch eine beidseitige Drehrichtung (Vorwärts und Rückwärts) realisiert werden. Des Weiteren kann der Rotor weitgehend frei positioniert werden, was zum Beispiel im Rahmen des Ein- bzw. Auslegens der Fluidleitung, insbesondere im Rahmen einer automatischen Ein- und Ausfädelfunktion der Fluidleitung, erforderlich/vorteilhaft ist. Gleichzeitig können unerwünschte negative Krafteinleitungen auf Zahnflanken eines mit der Antriebswelle wirkverbundenen Getriebes oder einer Getriebeeinheit gar vollständig eliminiert werden. Akustische Einflüsse können gegenüber dem Stand der Technik deutlich reduziert werden. Insgesamt bewirkt die Erfindung einen positiven Einfluss auf die Lebensdauer des Getriebes. Prinzipiell ist die Erfindung bei nahezu allen bekannten Peristaltikpumpen anwendbar, gleich ob der Rotor und die Antriebswelle fest oder lösbar miteinander gekoppelt sind. Sie ist außerdem bei Form-, Kraft wie auch Stoffschluss zwischen Rotor und Antriebswelle anwendbar.

Die Peristaltikpumpe der erfindungsgemäßen Vorrichtung kann in üblicher Weise ein definiertes Volumen eines Mediums fördern, wie zum Beispiel Blut oder Dialysefluid, von einer Niederdruckseite, in der Regel die arterielle Seite, auf eine Hochdruckseite, in der Regel die venöse Seite. Die elastische Fluidleitung wird in diese zwischen Rotor und der durch das Stützflächenmodul ausgebildeten Stützfläche schlaufenförmig eingelegt. Sie kann dabei insbesondere durch das Gehäusemodul geführt oder gehalten sein. Der Rotor und die die elastische Fluidleitung stützende Stützfläche sind derart ausgebildet und aufeinander abgestimmt, dass zwischen ihnen eine Förderstrecke ausgebildet ist. In deren Verlauf kommt es bei Rotation des Rotors um die Rotorachse zu Verformen und Abklemmen der elastisch verformbaren Fluidleitung. Der Rotor ist derart ausgebildet, dass die Fluidleitung nur punktuell oder abschnittsweise zusammengequetscht wird. Er kann zum Beispiel gegen die Fluidleitung vorgespannte und/oder gegenüber der Rotorachse relativpositionierbare Quetschelemente aufweisen. Die durch den Kontakt mit dem Rotor verursachte Quetschstelle wandert mit dem rotierenden Rotor mit und wird quasi durch die Fluidleitung von der Niederdruckseite zur Hochdruckseite bewegt. Infolge dessen wird Fluid in Förderrichtung aus der Fluidleitung herausgedrückt. Nachfließendes Fluid wird durch Niederdruck, insbesondere Vakuum, der aufgrund elastischer Rückformung der Fluidleitung nach der Verformung durch den Rotor entsteht, in die Leitung hineingezogen. Die elastisch verformbare Fluidleitung kann zum Beispiel ein Schlauch sein.

Mit der Erfindung können insbesondere die folgenden Vorteile erzielt werden:
- Durch die (winkel-/umlaufbegrenzte) Freilauffunktion in dem Formschluss der Koppelstruktur können die Anforderungen an eine beidseitige Drehrichtung erhalten werden;
- unerwünschte negative Krafteinleitungen insbesondere auf die Zahnflanken einer (vorgeschalteten) Getriebeeinheit können (komplett) eliminiert werden;
- durch die integrierte Freilauffunktion werden akustische Einflüsse (deutlich) reduziert;
- es kann ein positiver Einfluss auf die Lebensdauer des Getriebes genommen werden;
- es ist eine deutlich leisere Behandlung von Patienten möglich;
- er ist weniger Verschleiß am Getriebe zu verzeichnen.

Vorteilhafte Ausführungsformen der Erfindung sind in den Unteransprüchen beansprucht und werden nachfolgend näher erläutert.

Eine Ausführungsform der Peristaltikpumpe ist dadurch gekennzeichnet, dass der Rotor und die Antriebswelle lösbar miteinander gekoppelt sind. Dies ermöglicht eine besonders einfach Reinigung und Wartung der Pumpe. Des Weiteren kann die Fluidleitung einfach ausgewechselt werden.

Die erste Führungskontur kann entweder rotorseitig oder antriebswellenseitig ausgebildet sein. Sie kann insbesondere in Form einer Führungsfläche, zum Beispiel in Form einer ebenen Führungsfläche ausgebildet sein. Besonders einfach ist eine solche Führungsfläche auszubilden, indem zum Beispiel die Antriebswelle einfach parallel zu deren Rotationsachse abgeflacht ist. Es ist besonders vorteilhaft, wenn die Koppelstruktur zwei zueinander parallele ebene erste Führungskonturen auf einander diametral gegenüberliegenden Seiten der Rotationsachse aufweist. Dies ermöglicht eine gleichmäßige Drehmomentübertragung, insbesondere im Falle von wechselnden Drehrichtungen.

Die zweite Führungskontur ist zum Zusammenwirken mit der ersten Führungskontur der Koppelstruktur ausgebildet und bestimmt. Sie kann insbesondere aus zumindest zwei Führungsflächen gebildet sein, die jeweils parallel zur Rotationsachse und zueinander schräg angeordnet sind. Auch die zweite Führungskontur kann antriebswellenseitig oder rotorseitig vorliegen. Ist die erste Führungskontur beispielsweise rotorseitig ausgebildet, ist die zweite Führungskontur antriebswellenseitig ausgebildet und umgekehrt. Die erste und die zweite Führungskontur bewirken die Übertragung des Drehmoments zwischen der Antriebswelle und dem Rotor.

Nach einer weiteren Ausführungsform der Erfindung kann die zweite Führungskontur im Übergang zwischen deren beiden Führungsflächen gerundet ausgebildet sein. Alternativ kann die erste Führungskontur derart gerundet sein. Der Radius dieser Rundung kann in einem Bereich von ca. 5mm bis ca. 25mm, bevorzugter von ca. 10mm bis ca. 20mm liegen, noch bevorzugter ca. 15mm betragen. Eine solche gerundete Geometrie ermöglicht einen besonders weichen Übergang zwischen Vorlauf und Nachlauf bei einer Laständerung im Bereich des durch die Koppelstruktur zur Verfügung gestellten Spiels. Die erste Führungskontur kann quasi auf dem Radius der zweiten Führungskontur bei einer Relativrotation im Spielbereich abrollen und weich von einer ersten Relativlage (z.B. Gleichlauf) in eine zweite Relativlage (z.B. Vorlauf) wechseln. Dies ermöglicht im Ergebnis ein sanftes Wiedereingreifen der Antriebswelle in einen Drehmomentschluss mit der Koppelstruktur und damit mit dem Rotor. Es ist von besonderem Vorteil, wenn die Koppelstruktur nach einer Ausführungsform der Erfindung zwei derartige zweite Führungskonturen auf einander diametral gegenüberliegenden Seiten der Rotationsachse aufweist.

Die zweite Führungskontur ist gerundet ausgebildet, abschnittsweise oder auch vollständig. Die Rundung verläuft dabei insbesondere parallel zur Rotationsachse. Ihr Radius kann vorzugsweise in einem Bereich von ca. 5mm bis ca. 25mm, bevorzugter von ca. 10mm bis ca. 20mm liegen oder noch bevorzugter ca. 15mm betragen. Es ist von besonderem Vorteil, wenn die Koppelstruktur nach einer Ausführungsform der Erfindung zwei derartige zweite Führungskonturen auf einander diametral gegenüberliegenden Seiten der Rotationsachse aufweist.

Die erste(n) und zweite(n) Führungskonturen können besonders einfach ausgebildet sein, indem nach einer weiteren Ausführungsform der Erfindung die Antriebswelle oder der Rotor eine mittige Nut oder beidseitige Abflachungen aufweist. Dies ist robust, einfach herzustellen und kann besonders einfach eine Demontierbarkeit des Rotors von der Antriebswelle ermöglichen.

Eine Ausführungsform der Erfindung ist dadurch gekennzeichnet, dass der Rotor mittels eines Verriegelungselements mit der Antriebswelle koppelbar ist oder das Verriegelungselement aufweist, wobei die erste Führungskontur oder die zweite Führungskontur (bzw. die ersten Führungskonturen oder die zweiten Führungskonturen) an dem Verriegelungselement ausgebildet sind. Das Riegelelement kann zum einen dazu dienen, zwischen Rotor und Antriebswelle einen Drehmomentschluss auszubilden. Zum anderen kann es dazu dienen, den Rotor in auf der Antriebswelle positioniertem Zustand dort zu verriegeln, so dass ein versehentliches Lösen des Rotors während einer Behandlung ausgeschlossen werden kann.

Das Verriegelungselement kann insbesondere um eine Schwenkachse quer zur Rotationsachse schwenkbar am Rotor angeordnet sein. Es kann in eine den Rotor und die Antriebwelle über die Koppelstruktur miteinander koppelnde Verriegelungsstellung und in eine den Rotor und die Antriebswelle voneinander entkoppelnde Entriegelungsstellung positionierbar sein. Bei einer Pumpe mit einem derartigen Schnellverriegelungselement ist vorteilhaft, dass ein Lösen/Auswechseln des Rotors ohne Zuhilfenahme eines Werkzeugs/Schraubwerkzeugs manuell und unter geringem zeitlichem Aufwand erfolgen kann. Das Verrieglungselement kann insbesondere in der Entriegelungsstellung derart mit dem Rotor zusammenwirken, dass es als Handgriff für eine manuelle Betätigung bzw. einen manuellen Antrieb des Rotors verwendet werden kann. Dies ist insbesondere vorteilhaft im Falle von Stromausfällen, um eine fortwährende Pumpfunktion sicherstellen zu können. In der Entriegelungsstellung kann der Rotor von der Antriebswelle entkoppelt werden, so dass zum Beispiel die Fluidleitung ausgetauscht und/oder die Pumpe gut zugänglich gereinigt werden kann.

Man kann auch sagen, dass die Erfindung sich auf ein funktionsintegriertes Verriegelungselement einer lösbaren formschlüssigen Welle-Nabe-Verbindung eines Rotors mit einer Antriebswelle für den Rotor bezieht. Rotor und ggf. Antriebswelle können Bestandteile einer peristaltisch arbeitenden Rollenpumpe, insbesondere einer Schlauchpumpe für die Medizintechnik sein, die ihren bestimmungsgemäßen Einsatz insbesondere in der extrakorporalen Blutbehandlung finden kann. Der Rotor ermöglicht zusammen mit den elastischen Materialeigenschaften des Pumpensegmentes, welches schlaufenförmig gegen eine zylindrische Lauffläche des Pumpengehäuses eingelegt wird, eine Pumpfunktion, die eine Blutförderung zu einem Dialysator sicherstellt.

Die Erfindung wird im Folgenden anhand beispielhafter, nicht einschränkender und in den angehängten Figuren gezeigter Ausführungsformen näher erläutert. Dabei zeigt:
Figur 1 eine schematische Darstellung eines Ausschnitts einer Vorrichtung zur extrakorporalen Blutbehandlung,
Figur 2 eine Aufsicht auf ein Verrieglungselement mit einer erfindungsgemäßen Koppelstruktur,
Figur 3 eine weitere Aufsicht auf das Verrieglungselement der Figur 2,
Figur 4 einen vergrößerten Ausschnitt einer erfindungsgemäßen Koppelstruktur,
Figur 5 eine Unteransicht eines Rotors mit einer erfindungsgemäßen Koppelstruktur in einem ersten Betriebszustand,
Figur 6 eine Unteransicht des Rotors in einem zweiten Betriebszustand und
Figur 7 eine Unteransicht des Rotors in einem dritten Betriebszustand.

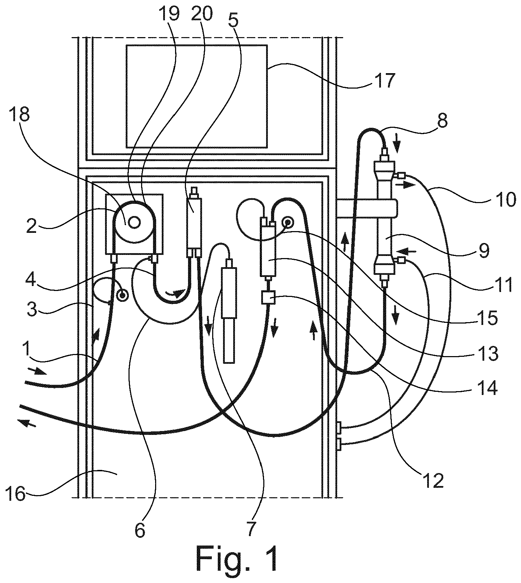

Figur 1 zeigt beispielhaft einen Ausschnitt einer Vorrichtung zur extrakorporalen Blutbehandlung nach der Erfindung. Gezeigt ist im Wesentlichen der gesamte extrakorporale Blutkreislauf der Vorrichtung. Dieser weist eine arterielle Blutleitung 1 auf, mittels der Blut von einem nicht gezeigten Patienten zu einer modular aufgebauten Peristaltikpumpe 2 der Behandlungsvorrichtung geführt wird. Vor der Peristaltikpumpe 2 ist ein arterieller Druckabnehmer 3 vorgesehen, mit dem der Druck vor der Peristaltikpumpe 2, also der niederdruckseitige Druck gemessen wird. Hochdruckseitig der Peristaltikpumpe 2 führt eine Hochdruckblutleitung 4 zu einem arteriellen Blutfänger 5. Unmittelbar am Ausgang der Peristaltikpumpe 2 kann mittels einer Zuleitung 6 und einer Pumpe 7 Zusatzstoff in das im System befindlichen Blut gegeben werden, z.B. Heparin zur Blutverdünnung.

Vom arteriellen Blutfänger 5 führt eine Leitung 8 unter Hochdruck stehendes aber noch unbehandeltes, mit Schlackenstoffen belastetes Blut zu einem Dialysator 9. Diesem wird eingangsseitig Dialysierflüssigkeit über eine Dialysierflüssigkeitszuleitung 10 zugeführt. Im Dialysator 9 wird Blut in bekannter Weise mittels der Dialysierflüssigkeit behandelt, z.B. gereinigt. Verbrauchte Dialysierflüssigkeit wird über eine Dialysierflüssigkeitsableitung 11 vom Dialysator 9 entfernt und einer nicht dargestellten Entsorgung oder Aufbereitung zugeführt. Behandeltes Blut wird mittels einer Blutableitung 12 vom Dialysator 9 zu einem venösen Luftfänger 13 geleitet, wo mittels einer Luftfalle 14 Luft abgeschieden wird. Am venösen Luftfänger 13 ist ein venöser Druckaufnehmer 15 vorgesehen, mit dem der venöse Druck, also der hochdruckseitige Druck, erfasst wird. Von der Luftfalle 14 wird behandeltes Blut über eine venöse Blutleitung 16 zurück zum Patienten geleitet. Dargestellt in Figur 1 ist auch eine Einheit 17 zur Überwachung und Steuerung der Vorrichtung. Die Vorrichtung zur extrakorporalen Blutbehandlung ist mit einem Gehäuse 100 gekapselt, das zumindest teilweise als Blechformteil ausgebildet ist.

Die Peristaltikpumpe 2 weist einen in Figur 1 angedeuteten Rotor 18 und ein ebenfalls angedeutetes Pumpengehäuse 19 mit einer Führungsfläche auf. Zwischen dem Rotor 18 und der Führungsfläche des Pumpengehäuses 19 ist eine elastisch verformbare Fluidleitung 20 angeordnet, die eingangsseitig mit der arteriellen Blutleitung 1 und ausgangsseitig mit der Hockdruckblutleitung 4 verbunden ist. Die Fluidleitung 20 wird durch die Wirkung des Rotors 18 zwischen diesem und der Führungsfläche des Pumpengehäuses 19 verformt und zusammengequetscht, derart, dass eine Fluidförderung von der arteriellen Blutleitung 1 zur Hochdruckblutleitung 4 bewirkt wird.

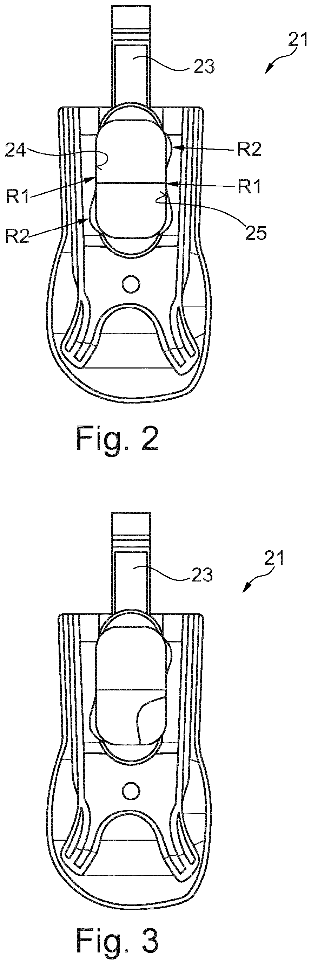

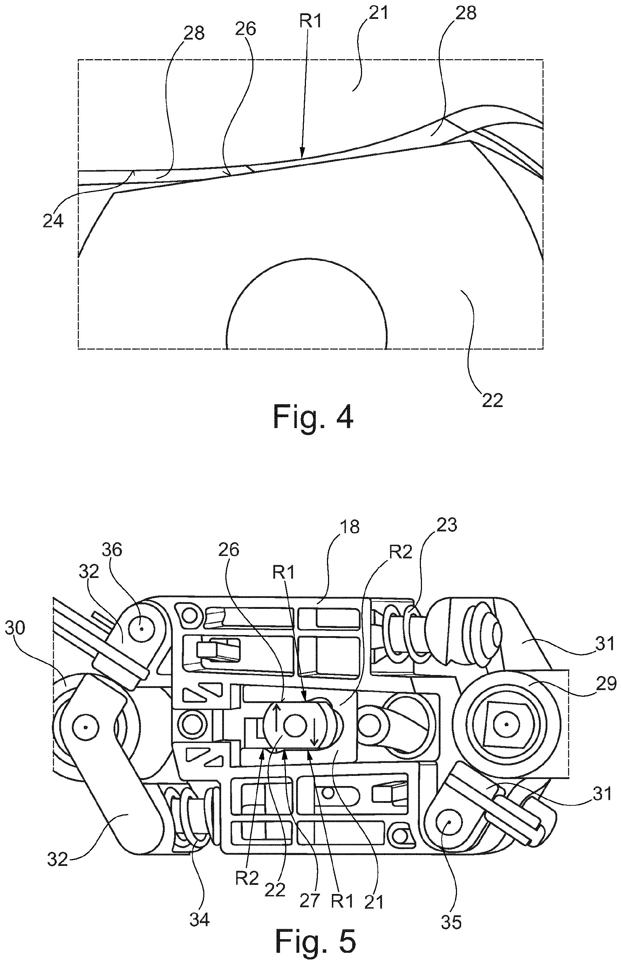

Figur 2 zeigt ein Verriegelungselement 21 zur Verriegelung des Rotors 18 auf einer in den Figuren 4 bis 7 erkennbaren Antriebswelle 22 der Dialysemaschine. Das Verriegelungselement 21 weist eine Schwenklasche 23 auf, mit der es schwenkbar am Rotor 18 festlegbar ist. In dem Verriegelungselement 21 ist eine Ausnehmung ausgebildet, deren Wände 24, 25 jeweils eine zweite Führungskontur im Sinne der Erfindung bilden.

Die Figuren 5 bis 7 zeigen die zweite Führungskontur 24, 25 des Verriegelungselements 21 in Eingriff mit der Antriebswelle 22. Diese ist beidseitig parallel zu ihrer Rotationsachse abgeflacht ausgebildet und weist einander diametral gegenüberliegend zwei erste Führungskonturen in Form von Abflachungen oder Führungsebenen 26, 27 auf. Diese Führungsebenen 26, 27 sind eben und zueinander parallel ausgebildet. Hingegen sind die Führungsflächen 24, 25 der ersten Führungskontur gerundet mit einem ersten Radius R1 und einem zweiten Radius R2 ausgebildet. Der erste Radius kann nach der Erfindung ca. 5mm bis ca. 25mm, bevorzugter von ca. 10mm bis ca. 20mm, noch bevorzugter von ca. 15mm betragen. Der zweite Radius beträgt vorzugsweise ca. 3mm. In den Figuren 5 bis 7 sind des Weiteren Anpressrollen 29, 30 des Rotors 18 gezeigt, die mit der Fluidleitung 20 zusammenwirken und diese zur Förderung von Fluid dadurch zusammenquetschen. Die Anpressrollen 29, 30 sind jeweils an einem Schwenkarm 31 bzw. 32 drehbar gelagert, der jeweils mit einer Druckfeder 33, 34 in eine Anlage an der Fluidleitung 20 vorgespannt sind. Die Schwenkarme 31, 32 sind jeweils um eine Schwenkachse 35, 36 schwenkbar.

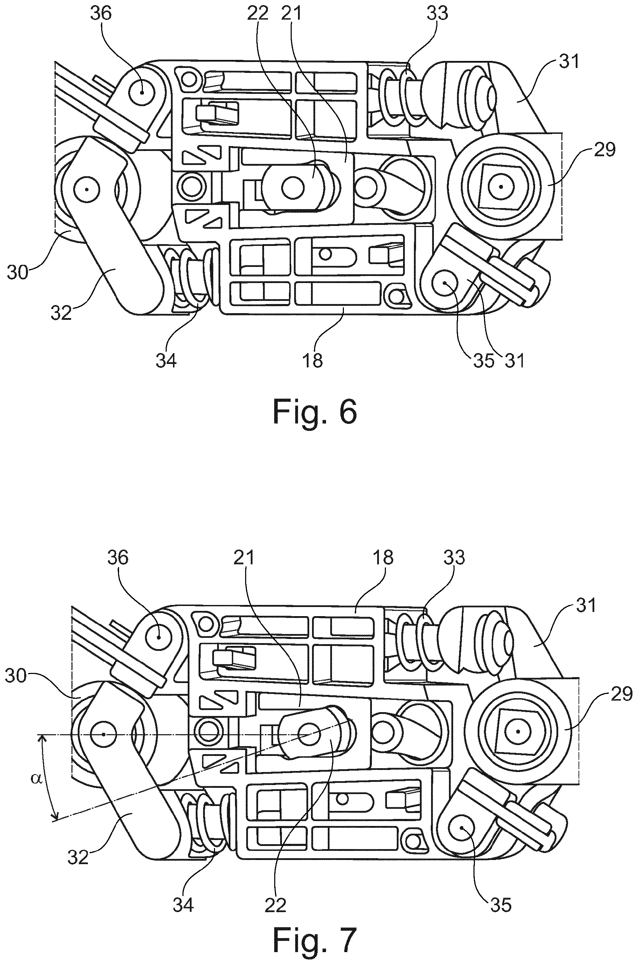

Befindet sich eine der Anpressrollen 29, 30 nicht mit der Fluidleitung 20 in quetschendem Eingriff, ist deren Schwenkarm 31 bzw. 32 durch die Wirkung der entsprechenden Druckfeder 33, 34 nach außen, also von der Antriebswelle 22 fort geschwenkt. Im Einlaufbereich gelangt die entsprechende Anpressrolle 29, 30 mit der Fluidleitung 20 in Kontakt und wird infolge des sich bei fortwährender Rotation verringernden Abstand zwischen der Anpressrolle 29, 30 zur Stützfläche in Richtung der Antriebswelle 22 nach innen verschwenkt. Nach Passieren des Einlaufabschnitts befindet sich die entsprechende Anpressrolle 29, 30 im Förderabschnitt der Fluidleitung 20 und quetscht dies fluidfördernd zusammen. Nach Durchlaufen des Förderabschnitts gelangt die entsprechende Anpressrolle 29, 30 in den Auslaufabschnitt, bei dessen Durchlaufen sich der radiale Abstand der Anpressrolle 29. 30 von der Führungsfläche wieder vergrößert und der entsprechende Schwenkarm 31, 32 infolge der geringer werdenden durch die Fluidleitung 20 ausgeübten Druckkraft wieder nach außen (fort von der Antriebswelle 22) schwenkt. Der maximale relative Schwenkwinkel α zwischen dem Rotor 18 und der Antriebswelle 22, der bei maximalen Vorlauf vorliegt, ist in Figur 7 eingezeichnet.

Figur 4 zeigt in einer Vergrößerung, dass zwischen den gerundeten Führungsflächen 24, 25 der zweiten Führungskontur und den ebenen Führungsebenen der ersten Führungskontur ein Spalt 28 gebildet ist, der ein Spiel in Rotationsrichtung bereitstellt. Es ist gut zu erkennen, dass die erste Führungskontur 26, 27 bei einer Relativrotation von Rotor 18 zur Antriebswelle 22 auf der zweiten Führungskontur 24, 25 abrollen kann. Dieser Abrollprozess oder die relative Rotation zwischen dem Rotor 18 und der Antriebswelle 22 wird besonders bei einem Vergleich der Figuren 5, 6 und 7 deutlich. In Figur 5 ist ein Betriebszustand gezeigt, bei dem die Antriebswelle 22 mit dem Rotor 18 in einer Arbeitsstellung oder Neutralstellung in Eingriff steht. Diese Stellung liegt vor, wenn sich keine der beiden Anpressrollen 29, 30 im Einlaufbereich oder im Auslaufbereich befindet. Befindet sich eine der Rollen 29, 30 im Bereich des Pumpenauslaufs (wo sich die Rolle von der Fluidleitung abhebt), wird der Rotor 18 durch die Anpresskraft der entsprechenden Druckfeder 33, 34 in Drehrichtung (in den Figuren im Uhrzeigersinn) beschleunigt und dreht sich daher zeitweise schneller als die Abtriebswelle 22. Dieser Zustand (Vorlauf) ist in den Figuren 6 und 7 dargestellt. Infolge des Spiels 28 wird die vorstehend beschriebene Relativpositionierung zwischen Rotor 18 und Antriebswelle 22 und werden damit die beschriebenen Schwankungen von Kräften und Momenten nicht auf die letztere übertragen.

## Patentansprüche

1. Peristaltikpumpe (2) einer Vorrichtung zur extrakorporalen Blutbehandlung, insbesondere einer Dialysemaschine, zum Fördern von Fluid in der Vorrichtung, welche Peristaltikpumpe (2) einen von einer Antriebswelle (22) drehangetriebenen Rotor (18) aufweist, der mit einer elastisch verformbaren Fluidleitung (20) eine Querschnittsverengung ausbildend zusammenwirkt, wobei
die Querschnittsverengung zur Fluidförderung durch Rotation des Rotors (18) entlang der Fluidleitung (20) deplatziert wird,
der Rotor (18) mit der Antriebswelle (22) mittels einer Koppelstruktur (24, 25, 26, 27) drehmomentübertragend gekoppelt ist,
die Koppelstruktur (24, 25, 26, 27) den Rotor (18) und die Antriebswelle (22) mit einem Spiel (28) in Rotationsrichtung zueinander koppelt, und
die Koppelstruktur (24, 25, 26, 27) zumindest eine parallel zur Rotationsachse ausgebildete ebene erste Führungskontur (26, 27) sowie zumindest eine zweite Führungskontur (24, 25) aufweist, welche zum Zusammenwirken mit der ersten Führungskontur (26, 27) ausgebildet und bestimmt ist,
**dadurch gekennzeichnet, dass**
die zweite Führungskontur (24, 25) abschnittsweise oder auch vollständig gerundet ausgebildet ist, um eine Abrollbewegung der ersten Führungskontur (26, 27) auf einem Radius der zweiten Führungskontur (24, 25) bei einer Relativrotation im Spielbereich zu bewirken.

2. Peristaltikpumpe (2) nach Anspruch 1, **dadurch gekennzeichnet, dass** der Rotor (18) und die Antriebswelle (22) lösbar miteinander gekoppelt sind.

3. Peristaltikpumpe (2) nach Anspruch 1 oder 2, **dadurch gekennzeichnet, dass** die Koppelstruktur (24, 25, 26, 27) zwei zueinander parallele ebene erste Führungskonturen (26, 27) auf einander diametral gegenüberliegenden Seiten der Rotationsachse aufweist.

4. Peristaltikpumpe nach einem der vorstehenden Ansprüche, **dadurch gekennzeichnet, dass** die zumindest eine zweite Führungskontur (24, 25) aus zumindest zwei Führungsflächen (24, 25) gebildet ist, die jeweils parallel zur Rotationsachse und zueinander schräg angeordnet sind.

5. Peristaltikpumpe (2) nach Anspruch 4, **dadurch gekennzeichnet, dass** die zweite Führungskontur (24, 25) im Übergang zwischen deren beiden Führungsflächen (24, 25) gerundet ausgebildet ist, vorzugsweise mit einem Radius (R1) von ca. 5mm bis ca. 25mm, bevorzugter von ca. 10mm bis ca. 20mm, noch bevorzugter von ca. 15mm.

6. Peristaltikpumpe (2) nach einem der vorstehenden Ansprüche, **dadurch gekennzeichnet, dass** die zweite Führungskontur (24, 25) gerundet ausgebildet ist, vorzugsweise mit einem Radius (R1) von ca. 5mm bis ca. 25mm, bevorzugter von ca. 10mm bis ca. 20mm, noch bevorzugter von ca. 15mm.

7. Peristaltikpumpe (2) nach einem der Ansprüche 4 bis 6, **dadurch gekennzeichnet, dass** die Koppelstruktur (24, 25, 26, 27) zwei derartige zweite Führungskonturen (24, 25) auf einander diametral gegenüberliegenden Seiten der Rotationsachse aufweist.

8. Peristaltikpumpe (2) nach einem der Ansprüche 1 bis 3, **dadurch gekennzeichnet, dass** die Antriebswelle (22) oder der Rotor (18) eine mittige Nut oder beidseitige Abflachungen aufweist.

9. Peristaltikpumpe (2) nach einem der vorstehenden Ansprüche, **dadurch gekennzeichnet, dass** der Rotor (18) mittels eines Verriegelungselements (21) mit der Antriebswelle (22) koppelbar ist oder das Verriegelungselement (21) aufweist, wobei die erste Führungskontur (26, 27) oder die zweite Führungskontur (24, 25) an dem Verriegelungselement (21) ausgebildet sind.

10. Peristaltikpumpe (2) nach einem der vorstehenden Ansprüche, **dadurch gekennzeichnet, dass** das Verriegelungselement (21) um eine Schwenkachse quer zur Rotationsachse schwenkbar am Rotor (18) angeordnet ist und in eine den Rotor (18) und die Antriebwelle (22) über die Koppelstruktur (24, 25, 26, 27) miteinander koppelnde Verriegelungsstellung und in eine den Rotor (18) und die Antriebswelle (22) voneinander entkoppelnde Entriegelungsstellung positionierbar ist.

## Claims

1. A peristaltic pump (2) of an apparatus for extracorporeal blood treatment, especially a dialysis machine, for conveying fluid in the apparatus, said peristaltic pump (2) including a rotor (18) driven to rotate by a drive shaft (22), said rotor (18) interacting with an elastically deformable fluid line (20) so as to form a cross-sectional constriction, wherein
said cross-sectional constriction is displaced along the fluid line (20) for conveying fluid by rotation of the rotor (18), and wherein the rotor (18) is coupled to the drive shaft (22) by means of a coupling structure (24, 25, 26, 27) so as to transmit a torque,
the coupling structure (24, 25, 26, 27) couples the rotor (18) and the drive shaft (22) having play (28) in the direction of rotation relative to each other, and
the coupling structure (24, 25, 26, 27) includes at least one flat first guiding contour (26, 27) formed in parallel to the axis of rotation as well as at least one second guiding contour (24, 25) formed and intended for interaction with the first guiding contour,
**characterized in that**
the second guiding contour (24, 25) is configured to be rounded in portions or completely to effect a roll-off movement of the first guiding contour (26, 27) on a radius of the second guiding contour (24, 25) during relative rotation within the play range.

2. The peristaltic pump (2) according to claim 1, **characterized in that** the rotor (18) and the drive shaft (22) are releasably coupled to each other.

3. The peristaltic pump (2) according to claim 1 or 2, **characterized in that** the coupling structure (24, 25, 26, 27) includes two flat first guiding contours (26, 27) parallel to each other on diametrically opposed sides of the axis of rotation.

4. The peristaltic pump (2) according to one of the preceding claims, **characterized in that** the at least one second guiding contour (24, 25) is formed of at least two guiding surfaces (24, 25) which are arranged in parallel to the axis of rotation and inclined relative to each other.

5. The peristaltic pump (2) according to claim 4, **characterized in that** the second guiding contour (24, 25) is configured to be rounded in the transition between the two guiding surfaces (24, 25) thereof, preferably having a radius (R1) from about 5 mm to about 25 mm, more preferred from about 10 mm to about 20 mm, even more preferred of about 15 mm.

6. The peristaltic pump (2) according to one of the preceding claims, **characterized in that** the second guiding contour (24, 25) is configured to be rounded, preferably having a radius (R1) from about 5 mm to about 25 mm, more preferred from about 10 mm to about 20 mm, even more preferred of about 15 mm.

7. The peristaltic pump (2) according to one of claims 4 to 6, **characterized in that** the coupling structure (24, 25, 26, 27) includes two second guiding contours (24, 25) of this type on diametrically opposed sides of the axis of rotation.

8. The peristaltic pump (2) according to one of claims 1 to 3, **characterized in that** the drive shaft (22) or the rotor (18) has a central groove or flattened portions on both sides.

9. The peristaltic pump (2) according to one of the preceding claims, **characterized in that** the rotor (18) is adapted to be coupled to the drive shaft (22) by means of a locking element (21) or includes the locking element (21), wherein the first guiding contour (26, 27) or the second guiding contour (24, 25) are formed on the locking element (21).

10. The peristaltic pump (2) according to one of the preceding claims, **characterized in that** the locking element (21) is arranged to be pivoting about a pivot axis transversely to the rotor axis on the rotor (18) and is adapted to be positioned into a locking position coupling the rotor (18) to the drive shaft (22) via the coupling structure (24, 25, 26, 27) and into an unlocking position uncoupling the rotor (18) from the drive shaft (22).

## Revendications

1. Pompe péristaltique (2) d'un dispositif destiné au traitement extracorporel du sang, en particulier une machine de dialyse, afin de transporter un fluide dans le dispositif, laquelle pompe péristaltique (2) comporte un rotor (18) qui est entraîné en rotation par un arbre d'entrainement (22), qui coopère avec une conduite fluidique (20) élastiquement déformable en formant un rétrécissement de section transversale, dans laquelle
le rétrécissement de section transversale est déplacé le long de la conduite fluidique (20) par la rotation du rotor (18) pour transporter le fluide,
le rotor (18) est couplé à l'arbre d'entrainement (22) au moyen d'une structure de couplage (24, 25, 26, 27) de manière à transmettre le couple,
la structure de couplage (24, 25, 26, 27) couple l'un à l'autre le rotor (18) et l'arbre d'entrainement (22) avec un jeu (28) dans la direction de rotation,
la structure de couplage (24, 25, 26, 27) comporte au moins un premier contour de guidage (26, 27) plan réalisé parallèlement à l'axe de rotation ainsi qu'au moins un deuxième contour de guidage (24, 25) qui est réalisé et déterminé pour coopérer avec le premier contour de guidage (26, 27),
**caractérisée en ce que**
le deuxième contour de guidage (24, 25) est réalisé partiellement ou complètement arrondi pour provoquer un mouvement de déroulement du premier contour de guidage (26, 27) sur un rayon du deuxième contour de guidage (24, 25) lors d'une rotation relative dans la zone de jeu.

2. Pompe péristaltique (2) selon la revendication 1, **caractérisée en ce que** le rotor (18) et l'arbre d'entrainement (22) sont couplés l'un à l'autre de manière amovible.

3. Pompe péristaltique (2) selon la revendication 1 ou 2, **caractérisée en ce que** la structure de couplage (24, 25, 26, 27) comporte deux premiers contours de guidage (26, 27) plans parallèles l'un à l'autre sur des côtés diamétralement opposés de l'axe de rotation.

4. Pompe péristaltique selon l'une quelconque des revendications précédentes, **caractérisée en ce que** l'au moins un deuxième contour de guidage (24, 25) est formé de deux surfaces de guidage (24, 25) qui sont agencées chacune parallèlement à l'axe de rotation et obliquement l'une par rapport à l'autre.

5. Pompe péristaltique (2) selon la revendication 4, **caractérisée en ce que** le deuxième contour de guidage (24, 25) est réalisé arrondi dans la transition entre ses deux surfaces de guidage (24, 25), de préférence avec un rayon (R1) de 5 mm environ à 25 mm environ, de manière plus préférée de 10 mm environ à 20 mm environ, de manière encore plus préférée de 15 mm environ.

6. Pompe péristaltique (2) selon l'une quelconque des revendications précédentes, **caractérisée en ce que** le deuxième contour de guidage (24, 25) est réalisé arrondi, de préférence avec un rayon (R1) de 5 mm environ à 25 mm environ, de manière plus préférée de 10 mm environ à 20 mm environ, de manière encore plus préférée de 15 mm environ.

7. Pompe péristaltique (2) selon l'une quelconque des revendications 4 à 6, **caractérisée en ce que** la structure de couplage (24, 25, 26, 27) comporte des deuxièmes contours de guidage (24, 25) de ce type sur des côtés diamétralement opposés de l'axe de rotation.

8. Pompe péristaltique (2) selon l'une quelconque des revendications 1 à 3, **caractérisée en ce que** l'arbre d'entrainement (22) ou le rotor (18) comporte une rainure médiane ou des aplatissements des deux côtés.

9. Pompe péristaltique (2) selon l'une quelconque des revendications précédentes, **caractérisée en ce que** le rotor (18) peut être couplé à l'arbre d'entrainement (22) au moyen d'un élément de verrouillage (21) ou comporte l'élément de verrouillage (21), le premier contour de guidage (26, 27) ou le deuxième contour de guidage (24, 25) étant réalisés au niveau de l'élément de verrouillage (21).

10. Pompe péristaltique (2) selon l'une quelconque des revendications précédentes, **caractérisée en ce que** l'élément de verrouillage (21) est agencé au niveau du rotor (18) de manière à pouvoir pivoter autour d'un axe de pivotement transversalement par rapport à l'axe de rotation et peut être mis dans une position verrouillée couplant l'un à l'autre le rotor (18) et l'arbre d'entrainement (22) par l'intermédiaire de la structure de couplage (24, 25, 26, 27) et dans une position déverrouillée découplant l'un de l'autre le rotor (18) et l'arbre d'entrainement (22).
